# EUROPEAN PATENT APPLICATION

(11) **EP 4 736 634 A1**
(43) Date of publication of application: **06.05.2026**
(21) Application number: 24210209.3
(22) Date of filing: 31.10.2024
(51) Int. Cl.: A01H 1/02, A01C 21/00

(54) **CONTROL OF HEADING IN CEREALS BY MOWING**

(71) Applicant: BASF SE, 67056 Ludwigshafen am Rhein (DE)
(72) Inventor: Moreau, Julien, 91630 Marolles en Hurepoix (FR); Delemme, Romain, 59310 Nomain (FR)
(74) Representative: Altmann Stößel Dick Patentanwälte PartG mbB

(57) **Abstract**

The present invention relates to a method for field cultivation comprising one or more areas with designated male cereal plants (male areas) and one or more areas with designated male-sterile female cereal plants (female areas), said method comprising the step of passing, during the stem elongation stage of the designated male plants, a cutting tool over at least part of each of said one or more male areas, but not over one or more female areas, to cut designated male cereal plants in said part of each of said one or more male areas. The present invention further relates to a field comprising one or more areas with designated male cereal plants and one or more areas with designated male-sterile female cereal plants, wherein the designated male plants in at least part of each male area were cut with a cutting tool during the stem elongation stage and wherein the designated female plants were not cut during the stem elongation stage. Moreover, the present invention relates to the use of a cutting tool for selectively cutting designated male cereal plants.

## Description

### Technical Field

The present invention relates to a method for field cultivation comprising one or more areas with designated male cereal plants (male areas) and one or more areas with designated female cereal plants (female areas), said method comprising the step of passing, during the stem elongation stage of the designated male plants, a cutting tool over at least a part of each of said one or more male areas, but not over one or more female areas, to cut designated male cereal plants in said part of each of said one or more male areas.. The present invention further relates to a field comprising one or more areas with designated male cereal plants and one or more areas with designated female cereal plants, wherein the designated male plants in at least part of each male area were cut with a cutting tool during the stem elongation stage and wherein the designated female plants were not cut during the stem elongation stage. Moreover, the present invention relates to the use of a cutting tool for selectively cutting designated male cereal plants.

### Background art

Hybrid cultivars with improved agronomic traits are widely used for the production of plants with increased yield. For wheat, the most widely grown crop in the world, a large scale adoption of the hybrid technology has yet to come. Wheat is a strict self-pollinating crop, with a very low probability of cross-pollination. The self-pollinating nature of wheat makes hybrid production difficult and labor-intensive, as it requires an efficient cross-pollination that overcomes the self-pollination. The same applies to other cereal plants, such as barley, rye, or triticale. Usually, the cross-pollination is achieved by using male-sterile maternal plants that are planted in close proximity to paternal plants. Then, the male plants are allowed to cross-pollinate the male sterile female plants and the female plants will produce hybrid seed. Male sterile plants can be generated, e.g., by using cytoplasmic or genic male sterility systems or by chemical agents that selectively interfere with pollen development (using a CHA or chemical hybridization agent). Hybrid seed production based on cytoplasmic male sterility (CMS) frequently uses a system of three different lines. The first line (line A) is the (cytoplasmic) male-sterile female parent. The second line (line B) is isogenic to line A, except that line B has fertile cytoplasm. Line B is frequently also referred to as "maintainer line" as the cross between line A and line B plants allows for maintaining the A line. The third line (line R, restorer line) provides the male parent for hybrid seed production. It carries one or more fertility-restoring (Rf) genes which are able to restore male fertility. For hybrid seed production, plants of line R are crossed with plants of line A to produce hybrid seed.

Genic male sterility (GMS), frequently also referred to as nuclear male sterility (NMS), can result from mutations in genes controlling pollen and/or stamen development. GMS is caused by nuclear genes alone, mostly by recessive alleles (ms). For wheat, the MS1, MS5, MS9, MS22, MS26, and MS45 have been described as male sterility genes. Male fertility restorer genes are capable of restoring the male sterility.

Nevertheless, cross-pollination of some cereal plants, such as of wheat or of barley, is still challenging.

One way to achieve cross-pollination is by interplanting male and female plants in a mixed stand within centimeters of each other in the same rows (WO 2012/038350 A1, WO2015/135940 A1). However, in such a system it is not possible to harvest only the seeds from female parents. The male parent will produce inbred seed due to self-pollination. Thus, the harvested seeds contain a mixture of hybrid seed and inbred seed. If cross-pollination is not good, then high amounts of selfed male seed will be present in the harvested hybrid seed. Since this may not comply with regulatory requirements and reduces product quality/purity, wheat hybrid seed is typically produced by sowing the male and female plants in separate areas (sometimes referred to as strip planting or "bay-planting system"). The male areas contain the male pollinator plants and are in separate rows from the female areas which contain the male sterile female plants. The areas are separated to such a distance that still allows the pollination of the male sterile female plants by the pollen of the male plants and that seeds from the female plants can be harvested separately. Accordingly, the harvested seeds consist essentially of hybrid seeds (with only a low proportion of inbred seeds).

Moreover, cross-pollination is challenging because wheat flowers for a short time only. Therefore, it is advantageous to have synchrony between the pollen shedding of the male parent and the flowering of the female parent. In crop species planted in the spring, synchronization, i.e. alignment of flower timing between two parents, can be achieved by seeding male and female parents on different dates. However, for wheat planted in the fall, i.e. winter wheat, synchronization cannot be achieved in this manner. To ensure successful pollen delivery from the male to the female, US2024/0016153 A1 suggests use of a topical treatment to slow growth rates in the male parent to delay heading and subsequent flowering. Also, a topical treatment is suggested to limit tillering thereby reducing late emerging spikes in the wheat canopy and shortening the male flowering window. Further, the seeding rate of the male plants in a seed production system is reduced to delay male flowering. It is said in US2024/0016153 A1 that when male flowering is delayed so that it minimizes overlap with flowering in the female plant, the opportunity for the male plants to self-fertilize is reduced, thereby reducing the production of male inbred seed and contamination of hybrid seed production.

In some countries, wheat is a crop utilized for green fodder as well as for the production of grain (frequently also referred to as "dual purpose wheat"). For this cultivation technique, plants are cut manually or mechanically early in the season and the forage is used to feed the cattle, and later the grain is harvested on re-grown crop. For harvesting green fodder, the aerial parts of the plant are cut mechanically or manually at certain stages of plant growth. In some Mediterranean countries, the wheat plants are not shortened by human intervention. Rather, they are clipped by grazing livestock ("Grain and Graze").

Various studies analyze the effect of the cutting on the dual purpose use of wheat.

Munsif et al. analyze the effect on sowing dates on the development of dual purpose wheat. Three sowing dates were tested. For each sowing date, the plants were cut 70 days after sowing with the help of sickle leaving about 4.0 cm stem portion above the ground. The authors conclude that late planting in mid-November resulted in short stature plants as compared to early planting in mid-October. With respect to the effect of the cutting on the development, the results are inconsistent. Whereas anthesis was delayed due to cutting in early planting dates, there were only slight differences in anthesis for later planting dates.

Li et al. analyze the regrowth pattern of winter wheat after mowing at seeding stage (Li et al., Int J Mol Sci. 2023 Oct 19;24(20):15353. doi: 10.3390/ijms242015353. PMID: 37895031; PMCID: PMC10607078). The plants were cut when the shoots had 5 to 6 tillers with a sickle, leaving approximately 2 cm of wheat stubble in the field.

Rossi et al. analyze the effect of grazing wheat on phenology and root growth (Plants (Basel). 2023 Jan 29;12(3):588. doi: 10.3390/plants12030588. PMID: 36771671; PMCID: PMC9921935). In order to simulate grazing wheat was clipped at 5 cm from the ground at the end of the tillering stage before the crops developed the first hollow stem. The final number of spikes was unaffected.

Tian et al. analyze the effect on yield of cutting dual purpose wheat at different developmental stages (Crop & Pasture Science, 2012, 63, 520-528 http://dx.doi.org/10.1071/CP12101). The plants were cut at ground level using hand shears. The authors observed delayed flowering depending on the developmental stage. Moreover, plants cut later than the developmental stage Z30 (Zadoks stage 30) had greatly reduced harvest index, tillers per m², and total N uptake. The authors conclude that cutting before stem elongation (GS 30) would be required to avoid large reductions in grain yield. While some published documents are discussed herein, this is not to be taken as any admission that any of these forms part of the common general knowledge of the person of ordinary skill in the art.

Despite the progress that has been made in the past in the field of hybrid cereals, such as wheat or barley, several challenges remain. For example, the low rate of cross-pollination is still a limiting factor for the generation of hybrid seeds. Therefore, means and methods are required to improve cross-pollination and thus to increase hybrid seed production. As outlined herein below, the present invention provides such means and methods.

### The Figures show:

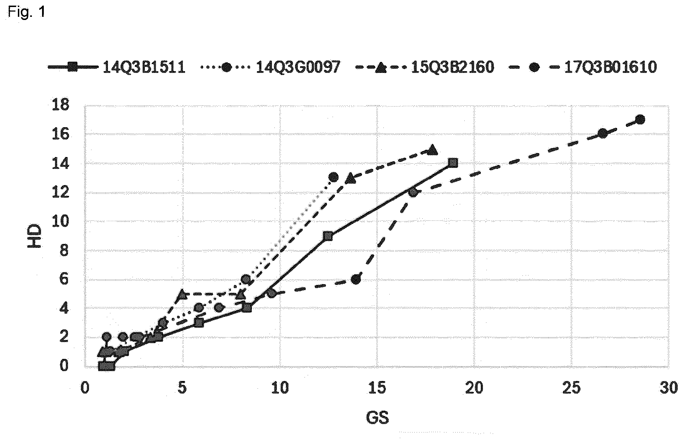

**Figure 1****:** Assessment of the effect of the mowing of plants on heading at different growth stages, compared to reference plants (not mowed). Four (4) different genotypes with different heading time were tested (i.e., line 14Q3B1511 (earliness score of 6.2), line 14Q3G0097 (earliness score of 5), line 15Q3B2160 (earliness score of 5.6), and line 17Q3B01610 (earliness score of 6.7)). The y axis shows the delay in heading (HD, in days), the x-axis shows the distance between the crown and the tip of the developing spike (ear tip) of the main tiller, which was measured prior to mowing (GS (for growth stage), in cm). The cutting height was set at 5 cm from the ground.

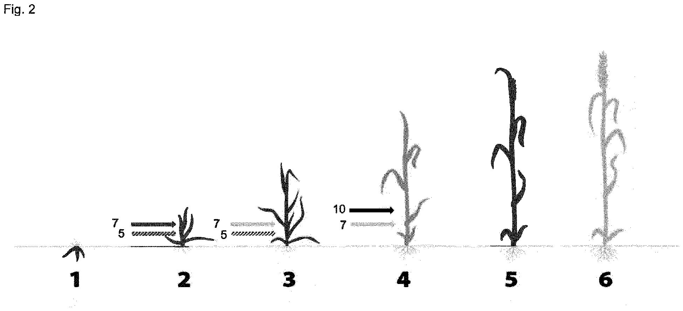

**Figure 2****:** Schematic overview of the experiment described in Example 2 (not drawn to scale). 14 different pollinators with different earliness classes (with a fertile cytoplasm, B lines) were grown on a field. In three different stages (about the one node stage (the plant with number 2 in Fig. 2), about the one to two nodes stage (the plant with number 3 in Fig. 2), and about more than two nodes stage (the plant with number 4 in Fig. 1)) the plants were cut with a lawn mower at one of two different cutting heights (as indicated by an arrow - the hatched arrow in Fig. 2 indicates a cut at 5 cm, the grey arrow indicates a cut at 7 cm, the black arrow indicates a cut at 10 cm)). Before cutting the plants with a lawn mower, the average aboveground height of the developing spike (i.e. ear) of the main tillers was determined by measuring the distance from developing spike tip to crown in a sample of plants (hereafter average aboveground height). The following cutting heights were applied: cutting at 5 cm or 7 cm in the plants when the average aboveground height was about 5 cm, cutting at 5 cm or 7 cm in the plants when the average aboveground height was about 7 cm, and cutting at 7 cm or 10 cm in the plants when the average aboveground height was about 10 cm.

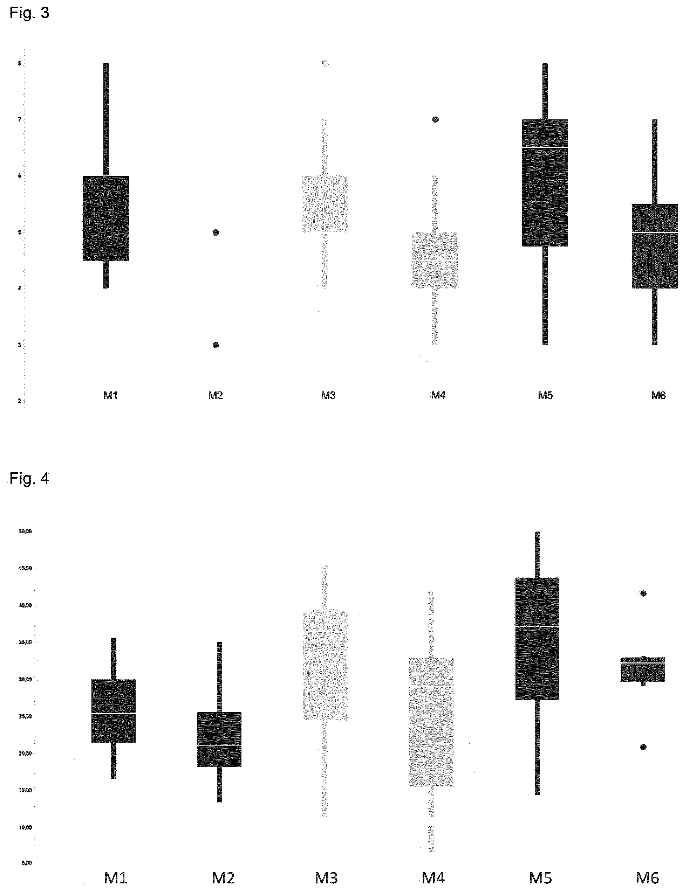

**Figure 3****:** Heading delay in cut wheat plants as compared to uncut wheat plants. The results are shown for i) plants that were cut at 5 cm (M1) or 7 cm (M2) when the average aboveground height was about 5 cm, ii) plants that were cut at 5 cm (M3) or 7 cm (M4) when the average aboveground height was about 7 cm, and, iii) plants that were cut at 7 cm (M5) or 10 cm (M6) when the average aboveground height was about 10 cm. In the x-axis the different stages (M1-M6) are shown, in the y-axis the delay in heading (in days) compared to uncut plants (values on the y-axis go from 2 at the bottom to 8 (days) at the top of Fig. 3, in steps of 1 day). The lighter bar indicates the median value.
**Figure 4****:** Spike reduction in cut wheat plants as compared to uncut wheat plants. The results are shown for i) plants that were cut at 5 cm (M1) or 7 cm (M2) when the average aboveground height was about 5 cm, ii) plants that were cut at 5 cm (M3) or 7 cm (M4) when the average aboveground height was about 7 cm, and, iii) plants that were cut at 7 cm (M5) or 10 cm (M6) when the average aboveground height was about 10 cm. In the x-axis the different stages (M1-M6) are shown, in the y-axis the reduction in the number of spikes (in %, values on the y-axis go from 5% at the bottom to 50% at the top of Fig. 4, in steps of 5) compared to uncut plants. The lighter bar indicates the median value.

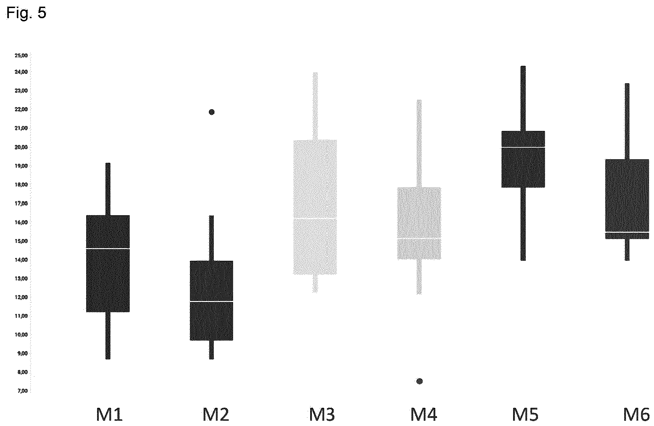

**Figure 5****:** Height reduction in cut wheat plants as compared to uncut wheat plants. The results are shown for i) plants that were cut at 5 cm (M1) or 7 cm (M2) when the average aboveground height was about 5 cm, ii) plants that were cut at 5 cm (M3) or 7 cm (M4) when the average aboveground height was about 7 cm, and, iii) plants that were cut at 7 cm (M5) or 10 cm (M6) when the average aboveground height was about 10 cm. In the x-axis the different stages (M1-M6) are shown, in the y-axis the reduction in plant height (in %, values on the y-axis go from 7% at the bottom to 25% at the top of Fig. 4, in steps of 1)) compared to uncut plants. The lighter bar indicates the median value.

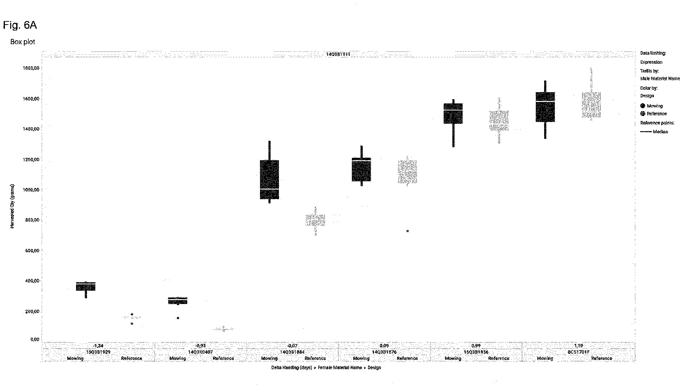

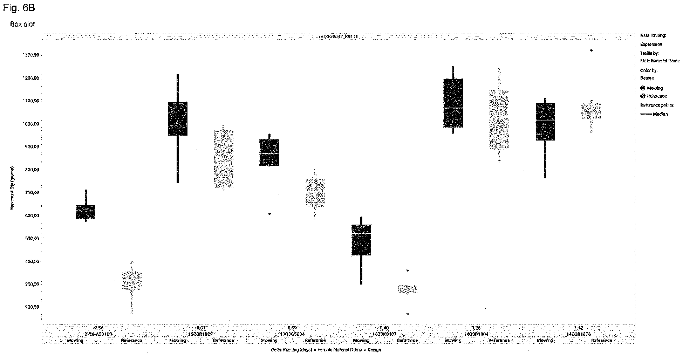

**Figure 6A** **and** **6B****:** Seed yield in female plants for crosses of different wheat lines in Example 3 (Fig. 6A with the male line 14Q3B1511, Fig. 6b with the male line 14Q3G0097_R0111). The male wheat plants were subjected to a cutting step. The seed yield of the female plants is shown as compared to a control (uncut male wheat plants). Especially, in the case of negative nicking (i.e. when the uncut male plants flower before the female plants), cutting the pollen donor plants increased the seed yield of the female plants. However, even in the case of positive nicking (i.e. when the uncut male plants flower after the female plants, mowing slightly increased or did not have any significant impact on the seed yield. The x-axis shows the different females from which seed yield was taken, with for each female at the left the results when the adjacent male was mowed ("Mowing") and at the right the results when the adjacent male was not mowed ("Reference"), the y-axis shows the harvested seed yield (in grams, values on the y-axis of Fig. 6A go from 0 at the bottom to 1800 at the top of Fig. 4, in steps of 200 (grams), values on the y-axis of Fig. 6B go from 200 at the bottom to 1300 at the top of Fig. 4, in steps of 100 (grams), with the lighter bar indicating the median value). The value indicated above each female name is the difference in heading with the male (e.g., -1.34 for the first female in Fig. 6A). If the female's heading date is before the male, the figure is positive. If the female's heading date is after the male, the value is negative.

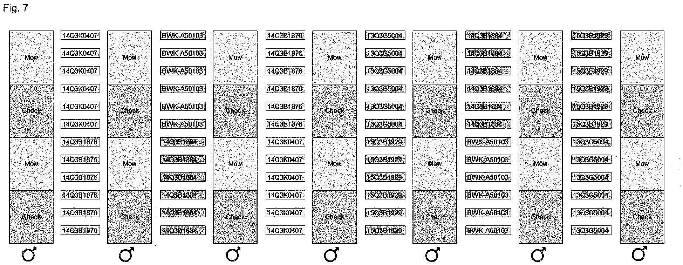

**Figure 7****:** field trial design of the experiment in Example 3, for the male plants tested in Fig. 6b, indicating the location of the cut ("Mow") and uncut ("Check") male areas, and the different female plant rows (not drawn to scale).

### Summary of the present invention

Advantageously, it has been found in the studies underlying the present invention that the cutting down of (male) cereal plants which should serve as pollen donator for the pollination of other plants at certain stages of the stem elongation stage allows for delaying the heading of the (male) plants by up to 6 days (Figure 1 and 3). In Example 1, four different wheat genotypes were cut at different growth stages to analyze the effect of the stage on the delay of heading. In Example 2, different cutting heights (low and high) were tested at three different stages (one node, two nodes and 2+ nodes) for line B wheat plants, i.e. plants that serve as pollen donator (Example 2). A delay of heading of at least 2 days was observed in all six experiments. On average, about 4 to 6 days of heading delay was observed. In general, the low cutting heights resulted in a longer delay of heading as compared to the high cutting heights (for all three tested stages, see Figure 3). Notably, the delay in heading was observed for different wheat cultivars, including wheat cultivars that flower early and that flower late. Also, it was found that to obtain a similar heading delay at a later stage compared to an earlier stage, a higher cutting height is to be used.

Moreover, a spike reduction of 22% to 35% (as compared to uncut plants) was observed. A similar spike reduction was observed for the low and high cutting height at the same growth stage. However, the cutting at the two earlier stages (1 node, 2 node) resulted in lower spike reduction as compared to cutting at the late stage (2+ node stage, Figure 4). Therefore, it is advantageous to cut the plants at the two earlier stages, although cutting at the later stage still results in sufficient spike formation. Since the male plants' key role is to provide pollen to the male-sterile female plants, the male seed yield is less of a concern.

Moreover, the effect of the cutting on the height of the plants was tested and a height reduction of about 12% to 20% was observed (Figure 5). It was observed that cutting taller plants resulted in a higher reduction of the height of the plants. Therefore, it would be advantageous to cut the plants at the two earlier stages (1 node, 2 node). Also, in one embodiment the male plants used are susceptible or responsive to GA (gibberellic acid) so that they increase in plant height after application of GA, and male plants that were cut can be made taller by spraying GA over the male plants. On the other hand, the female plants can be made shorter by applying a plant growth regulator (PGR) on the female plants or by using dwarf female plants. Even though taller male plants are considered better for pollination of the female plants, in the below examples it was found that cutting male plants, resulting in shorter plants compared to uncut male plants, did not significantly reduce hybrid seed yield (i.e., it improved yield or had no significant negative effect on yield.

In a further experiment, the effect of cutting the male plants on the seed yield of crosses between various wheat lines was tested (see Example 3). In these experiments, male and female parent combinations were planted next to each other, with some male/pollinator plant plots mowed, whereas other male/pollinator plots were not mowed (see Fig. 7 for field trial design), so as to compare the effect of the cutting of the male plants on the hybrid seed yield, when compared to the uncut male plants. The studies indicate that cutting the pollinators shifts male plant pollination and is beneficial for hybrid seed yield. Especially, in the case of negative nicking (i.e. when the uncut male plants flower before the female plants); cutting the pollen donor plants increased the seed yield of the female plants. However, even in the case of positive nicking (i.e. when the uncut male plants flower after the female plants), mowing slightly increased or did not have any impact on the hybrid seed yield. Thus, there was almost no downside/penalty on hybrid seed yield when using this method.

Cutting down the male plants (but not the female plants) is, in particular, advantageous, for row/strip planting of cereal plants such as wheat plants, i.e. for fields in which the male plants and female plants are grown in separate rows/areas. The cutting down approach is applicable for any cereal hybrid seed production (i.e. crosses of male x female plants) and for male-sterile plant seed production/maintenance (crosses between line A wheat plants and line B wheat, where the B is very similar to the A line but is fertile (near-isogenic)). Ideally, this is done with areas of pollinator plants (male parent line or B line) next to areas of male-sterile plants.

The present invention, thus, relates to a method for cultivating a field, said field comprising one or more areas with designated male cereal plants (male areas) and one or more areas with designated male-sterile female cereal plants (female areas). The method comprises the step of passing, during the stem elongation stage of the designated male plants, a cutting tool over at least part of each of said one or more male areas, but not over one or more female areas, to cut the designated male cereal plants in said part of each of said one or more male areas.

In a preferred embodiment, the cereal is wheat, barley, triticale or rye. In a particularly preferred embodiment, the cereal plants are wheat plants.

In a preferred embodiment, the method comprises:
a) providing a field comprising one or more areas with designated male cereal plants and one of more areas with designated male-sterile female cereal plants, wherein the plants are in the stem elongation stage, and
b) passing a cutting tool over at least part of each of said one or more male areas, but not over one or more male-sterile female areas, to cut the designated male cereal plants in said part of each of said one or more male areas.

The present invention further relates to a method for delaying heading, or expanding the pollination window, of male cereal plants, or for synchronizing heading or flowering of designated male cereal plants and designated (male-sterile) female cereal plants on a field comprising one or more areas with designated male cereal plants (male areas) and one or more areas with designated (male-sterile) female cereal plants (female areas). Typically, the method comprises the step of passing, during the stem elongation stage of the designated male plants, a cutting tool over at least part of each of said one or more male areas, but not over one or more female areas, to cut the designated male cereal plants in said part of each of said one or more male areas. In one embodiment, the above method is a method for delaying heading or flowering of said male cereal plants.

In a preferred embodiment, the method for delaying heading or flowering, or expanding the pollination window, of male cereal plants, or for synchronizing heading or flowering of designated male cereal plants and designated male-sterile female cereal plants comprises
a) providing a field comprising one or more areas with designated male cereal plants and one or more areas with designated male-sterile female cereal plants, wherein the plants are in the stem elongation stage, and
b) passing a cutting tool over at least part of each of said one or more male areas, but not over the one or more female areas, to cut the designated male cereal plants in said part of each of said one or more male areas, thereby delaying heading, or expanding the pollination window of male cereal plants, or synchronizing heading or flowering of the designated male cereal plants and designated female cereal plants. In one embodiment, the above method is a method for delaying flowering of said male cereal plants.

In a preferred embodiment, the method for delaying heading or flowering, or expanding the pollination window, of male cereal plants, or for synchronizing heading or flowering of male cereal plants and male-sterile female cereal plants comprises: passing a cutting tool over at least part of male plant rows/areas in a field, but not over the male-sterile female plant rows/areas in said field, to cut the male plants in said part, thereby delaying heading, or expanding the pollination window, of male cereal plants, or synchronizing heading or flowering of the male and female plants;
wherein said field comprises one or more rows/areas with designated male cereal plants and one or more rows/areas with designated male-sterile female cereal plants, and wherein said male plants are in the stem elongation stage. In one embodiment, the above method is a method for delaying flowering of said male cereal plants.

Heading, as used herein, refers to the stage when inflorescense/spikes emerge and start to be visible. Heading begins at Z50 when the first awns or the tip of the head become visible above the flag leaf collar, and ends at Z59 with full emergence of the head. Heading, as used herein, precedes and does not include the stage of flowering/anthesis (which starts at Z60, the beginning of pollination).

In a preferred embodiment of the above methods, said cutting tool, at least partially, removes the developing spikes of the main tillers from at least a portion of the designated male cereal plants.

The present invention further relates to a method to increase cross-pollination between designated male cereal plants and designated male-sterile female cereal plants on a field comprising one or more areas with designated male cereal plants (male areas) and one or more areas with designated male-sterile female cereal plants (female areas). Typically, the method comprises the step of passing, during the stem elongation stage of the designated male plants, a cutting tool over at least part of each of said one or more male areas, but not over one or more female areas, to cut the designated male cereal plants in said part of each of said one or more male areas. In one embodiment, no female plant is cut in said field.

Preferably, said method comprises:
a) providing a field comprising one or more areas with designated male cereal plants and one or more areas with designated male-sterile female cereal plants, wherein the plants are in the stem elongation stage, and
b) passing a cutting tool over at least part of each of said one or more male areas, but not over the one or more female areas, to cut the designated male cereal plants in said part of each of said one or more male areas, thereby increasing cross-pollination between the designated male cereal plants and designated female cereal plants.

In a preferred embodiment of the above method, said cutting tool, at least partially, removes the developing spikes of the main tillers from at least a portion of the designated male cereal plants, thereby increasing cross-pollination between the designated male cereal plants and designated female cereal plants.

The present invention further relates to a method for seed production, said method comprising
a) providing a field comprising one or more areas with designated male cereal plants and at and one or more areas with designated female cereal plants, wherein the plants are in the stem elongation stage,
b) passing a cutting tool over at least part of each of said one or more male areas, but not over the one or more female areas, to cut the designated male cereal plants in said part of each of said one or more male areas,
c) allowing the designated male and female cereal plants to grow, to cross-pollinate and, allowing at least the designated female cereal plant to develop seeds, and
d) harvesting seeds from the plants, in particular, harvesting the seeds from the designated female plants.

The present invention also relates to a method for improving hybrid seed production, said method comprising : passing a cutting tool over at least part of male plant rows/areas in a field, but not over the male-sterile female plant rows/areas in said field, to cut the male plants in said part, thereby improving hybrid seed production; wherein said field comprises one or more rows/areas with designated male cereal plants and one or more rows/areas with designated male-sterile female cereal plants, and wherein said male plants are in the stem elongation stage when cut.

The present invention further relates to a field comprising one or more areas with designated male cereal plants and one or more areas with designated male-sterile female cereal plants, wherein the designated male plants in at least part of each male area were cut with a cutting tool during the stem elongation stage and wherein the designated female plants were not cut during the stem elongation stage. In a preferred embodiment, the developing spike of the main tiller has been removed, by a cutting tool, from at least a portion of the designated male cereal plants, but not from the female cereal plants.

Moreover, the present invention relates to the use of a cutting tool for selectively cutting designated male cereal plants during the stem elongation stage on a field, said field comprising at least one area with designated male cereal plants and at least one area with designated female cereal plants. In one embodiment, said at least one male plant area is located next to said at least one female plant area, with only a narrow space between them (at most the distance needed to allow a tractor wheel to drive between said male and female area). Moreover, the present invention relates to the use of a cutting tool for selectively removing the developing spikes of the main tiller from designated male cereal plants that are grown on a field, said field comprising one or more areas with designated male cereal plants and one or more areas with designated female cereal plants, wherein the plants are in the stem elongation stage.

In one embodiment of the methods, use or field of the present invention, the designated male cereal plants are in the stem elongation stage. In particular, the designated male plants are in the one-node stage, two-node stage or 2+-node stage. In another embodiment of the method, use or field of the present invention, the designated male cereal plants are in the one-node stage or two-node stage.

In one embodiment of the methods of the present invention, the methods comprise, prior to the cutting step, the step of determining the average aboveground height of the developing spikes of the main tillers of the designated male cereal plants to set the height at which the cutting tool cuts the designated male cereal plants.

Preferably, the average aboveground height of the developing spikes of the main tillers of the male plants grown on the field is determined to set (or confirm) the cutting height at which the cutting tool cuts the designated male cereal plants. The average aboveground height of the plants on the field can be determined for a sample, i.e. a random sample of designated male plants grown on the field.

Preferably, the determination of the average aboveground height of the developing spikes of the main tillers of the male plants comprises:
i) measuring the distance from a) the lower end of the crown to b) the upper end (tip) of the developing spike on the main tiller in at least five randomly selected male plants, in particular in at least ten randomly selected male plants, and
ii) determining the average aboveground height by calculating the average of the distances measured in step i).

Hence, the "aboveground height of the developing spikes", as used herein, refers to the average distance as measured between the lower end of the crown and the tip of the developing spike on the main tiller in a random sample of (e.g., 5 or 10) plants in a field.

More preferably, the average aboveground height is determined by carrying out the following steps:
i) harvesting at least five male plants, in particular in at least ten male plants,
ii) making a transversal cut through the main stem of each of the plants harvested in step i),
iii) measuring, in the cuts obtained in step ii), the distance from a) the lower end of the crown to b) the upper end (tip) of the developing spike for each of the plants, and
iv) determining the average aboveground height by calculating the average of the distances measured in step iii).

In a preferred embodiment of the methods, the field or the use of the present invention, the cutting tool cuts (or has cut) the designated male cereal plants at a height which is equal to or below the average aboveground height of the developing spikes of the main tillers of the male plants. Preferably, the cutting tool cuts the designated male cereal plants (over which it is passed) at a height which is up to 25% lower, such as 10% lower than the average aboveground height of the developing spikes of the main tillers of the male plants. Also preferably, the cutting tool cuts the designated male cereal plants at a height which is higher than the average height of the developing spikes of the secondary tillers of the male plants (so that most of the developing spikes on the secondary tillers are not cut). Thus, the cutting height of the cutting tool is, preferably, below the average aboveground height of the developing spikes of the main tillers, but above the average height of the developing spikes of the secondary tillers. Accordingly, the cutting tool, preferably, removes most of the developing spikes on the main tillers of the plants over which the tool is passed, but does not remove most of the developing spikes of the secondary tillers. As used herein, the height of the developing spikes of the secondary tillers, refers to the height from the soil to the tip of the highest secondary spikes, and preferably the cutting tool as used herein cuts most of the developing spikes on the main tillers, but does not cut most developing spikes on the secondary tillers.

Cutting most developing spikes on the main tiller of the male plants in the male area that is cut, as used herein, refers to cutting (removing) more than 50 % of the developing spikes on the main tillers in said male area that was cut (as can be checked by cutting a test area of 1-3 m that was cut with the cutting device, or a test area of 1, 2, or 3 m²), in a field planted with designated male cereal plants and designated male-sterile female cereal plants, or cutting (removing) more than 60 %, more than 65 %, more than 70 % or more than 75 % of the developing spikes on the main tillers in said male area that was cut. Not cutting most developing spikes on the secondary tillers of the male plants in the male area that is cut, as used herein, refers to cutting (removing) less than 50 % of the developing spikes on the secondary tillers in said male area that was cut (as can be checked by cutting a test area of 1-3 m that was cut with the cutting device, or a test area of 1, 2, or 3 m²), in a field planted with designated male cereal plants and designated male-sterile female cereal plants, or cutting (removing) less than 40 %, less than 35 %, less than 30 % or less than 25 % of the developing spikes on the secondary tillers in said male area that was cut.

In a preferred embodiment of the methods, the field or the use of the present invention, the average aboveground height of the developing spikes of the main tillers of the designated male cereal plants is at least 4 cm, such as 4 to 15 cm. Preferably, the cutting height is from 4 cm to 12 cm.

In an embodiment of the methods, the field or the use of the present invention, the field comprises one area with designated male-sterile female cereal plants and one area with designated male cereal plants.

In an embodiment of the methods, the field or the use of the present invention, the field comprises at least two areas with designated male-sterile female cereal plants (female areas) and at least two areas with designated male cereal plants (male areas), wherein the male areas and the female areas alternate.

In a preferred embodiment of the methods, the field or the use of the present invention, the field comprises at least three areas with designated male-sterile female cereal plants (female areas) and at least three areas with designated male cereal plants (male areas), wherein the male areas and the female areas alternate.

In a preferred embodiment of the methods, the field or the use of the present invention, the male areas are physically separated from the female areas. The distance between the female area(s) and the adjacent male area(s) should be a distance that allows for pollinating the female plants by pollen of the male plants and for harvesting the seeds from the female plants separately.

In a preferred embodiment of the methods, the field or the use of the present invention, the distance between the female areas and the adjacent male areas is from 10 cm to 70 cm, such as from 20 to 50 cm, in particular from 20 to 40 cm.

In a preferred embodiment of the methods, the use, or the field the cutting tool is passed (or has been passed) over the complete area of each of said one or more male areas, or over only a part of each of said one or more male areas. Preferably, said part is an area of 20% to 80% of the complete area of the at least one male area. For example, said part is an area of 30% to 70%, such as 40 to 60 %, or 50 %, of the complete area of the at least one male area. Preferably, the cutting tool is passed (or has been passed) over the part of said area which is adjacent to a female area. In other words, the tool has not been passed over the male plants that are more distant from the female area. Thus, the cutting tool is passed only over a part of each of said one male areas, wherein said part is preferably the part adjacent to a female area.

In a preferred embodiment of the methods, the use, or the field of the present invention, the debris that results from the cutting step is not removed (or has not been removed) from the field.

In a preferred embodiment of the methods, the use, or the field of the present invention, one or more of the following effects is achieved:
- the heading or flowering of designated male cereal plants is delayed as compared to corresponding control plants that were not subjected to the cutting step of the present invention,
- the heading or flowering of the designated male cereal plants and the designated male cereal plants is more synchronized as compared to corresponding control plants that were not subjected to the cutting step of the present invention, and/or
- the hybrid seed yield of said field is increased as compared to a corresponding control field that was not subjected to the cutting step of the present invention.

In a preferred embodiment of the methods, the use, or the field of the present invention, the cereal is a winter cereal. Accordingly, the designated male plants and designated female plants are derived from seeds that were sown in the fall.

In an embodiment, the winter cereal is selected from winter wheat, winter barley, winter triticale and winter rye. In a particularly preferred embodiment, the cereal is winter wheat.

In one embodiment, the ears of the designated male plants are removed, or the designated male plants are destroyed, after pollination (before they set seed), so that only the hybrid seed on the female plant rows can be harvested from the field.

In a preferred embodiment of the methods, the use, or the field of the present invention, the cutting tool is a mowing machine or a reaping machine in particular a mechanical mowing machine or a mechanical reaping machine. In a preferred embodiment, the mowing machine is a lawn mower. Optionally, the cutting tool is capable of mulching the debris. If so, it is envisaged that the debris is mulched.

In a preferred embodiment of the methods, the use, or the field of the present invention, the female cereal plants are dwarf plants. Alternatively, or additionally, the designated female cereal plants are treated with a plant growth regulator (such as a plant growth regulator with trinexapac-ethyl as the active ingredient (e.g., Prodax ^{®}, Palisade^{®} EC, or Moddus^{®}, T-Nex^{®}, or PrimoMAXX)) that reduces the height of said plants.

In a preferred embodiment of the methods, the use, or the field of the present invention, the designated male cereal plants are treated with a plant growth stimulant (such as gibberellic acid) that increases the height of said plants. Alternatively, or additionally, the designated male cereal plants are genetically taller than the designated female cereal plants.

In a preferred embodiment of the methods, the use, or the field of the present invention, the designated female cereal plants are (female) male sterile plants.

In a preferred embodiment, the (female) male sterile plants are cytoplasmic male sterile (CMS) plants. In this case, the designated male cereal plants, typically, have a fertile cytoplasm. In an embodiment, the male plants and the female cytoplasmic male sterile (CMS) plants have an identical nuclear genotype or a nearly identical nuclear genotype (such as a sterile CMS line and the fertile maintainer line thereof). In another embodiment, the designated male cereal plants and the designated female cytoplasmic male sterile (CMS) plants are genetically diverse. In this embodiment, the designated male cereal plants comprise one or more fertility-restoring (Rf) genes which are able to restore male fertility of the plants grown from the seeds produced by the designated female cereal plants.

In another preferred embodiment, the female male sterile plants are genetic male sterile (GMS) plants. Examples of genetic male sterility systems that can be used in the current invention are those described in WO2024030797, WO2020056259, WO2019118342, WO2019043082, WO2023009993, WO2019165199, or WO20180022410, preferably a non-transgenic 2-line hybrid system, such as a GMS based on mutants in the cereal genes MS1, MS5, MS45, MS9, MS22, or MS26 which cause male-sterility (and can be restored by any normal wheat).

In yet another preferred embodiment, the female male sterile plants are conditionally male sterile plants that are obtained by treating the designated female plants with one or more chemical hybridization agents (CHA, such as sintofen or clofencet).

### Detailed overview on the present invention / Definitions

### The field

The methods of the present invention, i.e. i) the method for field cultivation, ii) the method for delaying heading, or expanding the pollination window, of male cereal plants, or for synchronizing heading/flowering of designated male cereal plants and designated female cereal plants, iii) the method for synchronizing heading/flowering of designated male cereal plants and designated female cereal plants, and iv) the method for seed production comprises the step of passing a cutting tool over the one or more areas with designated male plants which are grown on a field comprising one or more areas with designated male cereal plants and one or more areas with designated male-sterile female cereal plants.

Accordingly, the field in accordance with the present invention comprises one or more areas with designated male cereal plants and one or more areas with designated male-sterile female cereal plants.

The cereal is, preferably, wheat, barley, triticale or rye. Thus, the plants are wheat, barley, triticale or rye plants.

In an embodiment, the field comprises one or more areas with designated male wheat plants and one or more areas with designated female wheat plants.

In another embodiment, the field comprises one or more areas with designated male barley plants and one or more areas with designated female barley plants.

In another embodiment, the field comprises one or more areas with designated male rye plants and one or more areas with designated female rye plants.

In another embodiment, the field comprises one or more areas with designated male triticale plants and one or more areas with designated female triticale plants.

In a preferred embodiment, the plants are wheat plants. The term "wheat", as used herein, typically, refers to plants from the genus *Triticum,* including but not limited to common/bread wheat (*Triticum aestivum* or *T. aestivum*), emmer wheat (T. *dicoccum*), einkorn wheat (T. *monococcum*), durum wheat (*T*. *durum*), khorasan wheat (*T*. *turanicum*), or spelt wheat (T. *spelta*), specifically hexaploid *T. aestivum* or *T. spelta,* and tetraploid *T. durum,* including wheat referred to as hard or soft wheat (based on endosperm texture), winter or spring wheat (based on sowing season), and red or white wheat (based on seed coat color). In particular, the wheat *is Triticum aestivum.*

In preferred embodiment, the cereal is a winter cereal, in particular winter wheat. Winter wheat (typically *Triticum aestivum*) is wheat that is planted, i.e. sown, in the fall, for example from September to November in the Northern hemisphere. After germination, winter wheat develops into young plants that remain in the vegetative phase in the winter and resume growth in early spring. For developing the ear, winter wheat requires a prolonged period of cold, for example a period of 30 to 60 days of cold winter temperatures (0 to 5 °C).

In an embodiment, the methods of the present invention, thus comprises the step of planting the designated male cereal plants (such as wheat plants) and designated female cereal plants (such as wheat plants) in the fall, for example in September, October or November (in the Northern hemisphere). Thus, the seeds for the plants are typically sown in the fall.

Typically, the seeds for designated male cereal plants and the seeds for designated female cereal plants are sown at the same time, typically, within a period of 24 hours. However, it is also envisaged that they are sown not more than 7 days apart, for example not more than 3 days apart.

In accordance with the present invention, the field comprises a population of designated male cereal plants (herein also referred to as "male plants") and a population of designated male-sterile female cereal plants (herein also referred to as "female plants"). With respect to the male plants, the term "designated", typically, means that the male plants were planted with the intention to serve as pollen donator for the female plants. With respect to the female plants, the term "designated", typically, means that the female plants were planted with the intention that they serve as pollen acceptor of the pollen provided by the male plants.

In order to separate the male from the female plants, the male plants and the female plants are, preferably, grown in separate areas on said field. The one or more male areas contain the male pollinator plants and these are separated, i.e. physically separated, from the female areas which contain the female plants. Preferably, no plants are planted in the spaces between the male areas and the female areas.

The field in accordance with the present invention, thus comprises one or more female areas and one or more male areas. The area could be any area deemed appropriate. Preferably, the area is one or more rows, a strip or a block. In an embodiment, the area is a block. In another embodiment, the area is a strip. In yet another embodiment, the area is a combination of several rows in a strip. The individual areas on the field may have any size deemed appropriate. However, it is envisaged that essentially all plants in the male areas are, in principle capable of pollinating female plants in the adjacent female area(s).

Preferably, the male areas are separated from the female areas at such a distance which allows for pollinating the female plants by pollen of the male plants and which allows that the seeds produced in the female areas can be harvested without being contaminated by seeds produced by the male plants. For example, the seeds harvested from the at least one female block or from the at least one female area will contain at least 95% of seeds that result from cross-pollination.

In a preferred embodiment, the field comprises at least three areas with designated female wheat parent plants (female areas) and at least three areas with designated male wheat parent plants (male areas). Preferably, the male areas and the female areas alternate. Thus, the male plants may be planted in areas alternating with areas of the female plants.

It will be understood by the skilled person that essentially all plants in the female areas are (designated) female plants. However, a small percentage of male plants may be present. Preferably, however, at least 85% of the wheat plants present in the individual "female" areas are (designated) female plants. In particular, at least 90 %, at least 95 %, at least 96%, at least 97 %, at least 98 % or at least 99% of the wheat plants present in the individual "female" areas are (designated) female plants. In particular, all wheat plants present in a "female" area are (designated) female plants.

Similarly, it is envisaged that essentially all plants in the male areas are male plants. However, the male areas may contain a larger percentage of female plants. Preferably, however, at least 90 % or at least 95%, such as at least 96%, at least 97 %, at least 98 % or at least 99% of the wheat plants present in the individual "male" areas are (designated) male plants. In particular, all wheat plants present in a "male" area are (designated) male plants.

In a preferred embodiment, the field contains at least 1000 (male and female) plants, or each area, i.e. each male and each female area, on the field contains at least 1000 plants.

### Developmental stage of the plants to be cut

Typically, the male and female cereal plants on the field of the present invention are in the stem elongation stage. Preferably, at least the designated male plants are in the stem elongation stage.

For determining the stage of the plants in the field, for example of the male plants, the decimal (or 'Zadoks') growth stage code is applied herein, in particular the code published by Tottman, DR & Broad, H (1987. The decimal code for the growth stages of cereals, with illustrations. Annals of Applied Biology 110, 441-454). In order to assess the growth stage of plants grown on a field (or in an area) as a whole, the growth stage is, typically,determined for an odd number of plants grown on the field (such as 5, 7 or 9 plants). The median stage indicates the stage of the plants as a whole. Thus, the stage of the plants as referred to herein is typically the median stage. In particular, it is the median stage of the designated male plants.

The stem elongation stage as used herein encompasses Zadoks stages Z30, Z31, Z32, Z33, Z34, Z35, Z35, Z37 and Z39 (according to Zadoks). Thus, the designated male plants that are cut are typically in stage Z30, Z31, Z32, Z33, Z34, Z35, Z36, Z37 or Z39. Preferably, however, the plants are in Z31, Z32 or Z33, such as in stage Z31. Thus, the plants are, typically, in stage Z31 or Z32.

Preferably, at the time of the cutting, the plants in the field, in particular the designated male plants, are in the one-node-stage (Z31), two-node-stage (Z32) or three-node-stage or 2+-node (Z33). More preferably, the plants are in the one-node or two-node stage, i.e. in Zadoks stage Z31 or Z32. Most preferably, the plants are in the one node stage, i.e. in Zadoks stage Z31.

Determination of the cereal growth stages Z31 and Z32 is described by the Agriculture and Horticulture Development Board on the world wide web at: ahdb.org.uk/knowledge-library/the-growth-stages-of-cereals (see description of GS31 and GS32).

### The cutting step

The methods of the present invention comprise the passing of a cutting tool over at least part of the area or areas with the designated male cereal plants to cut the designated male cereal plants. However, the cutting tool is not passed over the area or areas with the designated female cereal plants. Accordingly, the female plants are not cut during the stem elongation stage.

Preferably, the cutting step allows for removing the developing spikes of the main tillers from at least a portion of the designated male cereal plants (over which the tool is passed). Accordingly, the cutting step, preferably, comprises passing a cutting tool, at least partially, over the area or areas with the designated male cereal plants, but not over the designated female cereal plants, to cut at least one/some male plants, such as to cut one or more male plant rows. Preferably, the cutting step removes the developing spikes of the main tillers from at least a portion of the designated male cereal plants. Accordingly, the cutting tool, preferably, cuts the designated male cereal plants (over which it is passed) at a height which is at or below the average aboveground height of the developing spikes of the main tillers of the male plants. As the plants grown on the field might differ in their height, such a cutting height would allow for removing the developing spikes of the main tillers from at least a portion of the designated male cereal plants over which the tool is passed. Preferably, said portion is a portion of least 30%, more preferably, of at least 60%, and most preferably, of at least 80% of the male plants of the male plants over which the tool is passed.

The "spike" is frequently also referred to as "ear" or head. It is originally formed as a microscopic structure between Zadoks stages 14 and 16 (Z14 and Z16). During stem elongation, the "spike" (or "ear") is pushed to the top of the crop canopy.

The cutting tool to be applied in the cutting step, preferably has a preselected cutting height, i.e. a cutting height that allows for cutting the designated male plants at a preselected height. The cutting tool preferably has an adjustable cutting height.

In a preferred embodiment of the present invention, the cutting height is a height which is equal to or slightly below the average aboveground height of the developing spikes of the main tillers. In a preferred embodiment of the present invention, the cutting height is up to 25% lower, such as up to 10% lower than or equal to the average aboveground height of the developing spikes of the main tillers. Such cutting heights allow for keeping a sufficient number of developing spikes of the secondary tillers. The average aboveground height of the developing spikes of the main tillers of the male plants can be determined as described herein. Based on the determined height, the cutting height of the cutting tool can be set.

Further, it is envisaged that the developing spikes of the secondary tillers of the male plants are not removed. Accordingly, it envisaged to cut plants at a height which is at or below the average aboveground height of the developing spikes of the main tillers of the male plants, but is higher than the average height of the developing spikes of the secondary tillers of the male plants. Thus, the cutting height of the cutting tool is below the average aboveground height of the developing spikes of the main tillers, but above the average height of the developing spikes of the secondary tillers. Accordingly, the cutting tool removes most of the developing spikes on the main tillers of the plants over which the tool is passed, but does not remove most of said developing spikes of the secondary tillers.

As will be understood by the skilled person, this approach might remove a portion of the developing spikes of the secondary tillers of the male plants because the plants do not grow in a uniform manner. However, the ratio of the number of the developing spikes of the main tillers of the male plants to the number of the developing spikes of the secondary tillers of the female plants will be decreased.

In one embodiment of the present invention, the cutting tool is passed over the complete area of each of said one or more male areas. Thus, the male area(s) may be moved completely. In another embodiment, the cutting tool is passed only over a part of the male block(s) or area(s). Thus, the male area(s) may be only moved partially. This allows for increasing the duration of the time period in which the male plants produce pollen. Preferably, the part (over which the cutting tool is passed) is an area of 20% to 80% of the complete area of the at least one male area. For example, said part is an area of 30% to 70% of the complete area of the at least one male area, such as 40 to 60 %, or 50 %. In case the cutting tool is passed only over a part of the male area(s) or block(s), it is, in particular, envisaged that said part is the part of said male area(s) which is adjacent to a female area. In other words, the cutting tool is passed over the part or part(s) which has (or have) the closest distance to the neighboring female area.

The cutting tool may be any tool that is deemed appropriate for cutting the plants that allows for cutting the plants at the desired height. Thus, the cutting tool, preferably, allows for the cutting at a low height, such as at heights of 4 to 15 cm.

In one embodiment, the cutting is tested in a test area (such as cutting 1-3 m with the cutting device, or cutting 1-3 m²) on the male area of a field, by setting the cutting height at the average aboveground height of the developing spike on the main tillers, as measured in a sample of 5-10 plants (ideally randomly taken from different parts of said male plant area), and then checking if most of the developing spikes on the main tillers were cut (and not most of the developing tillers on the secondary tillers) - if that is the case, the cutting height can be applied to the entire field. If the cutting did not remove sufficient developing spikes on the main tillers, the cutting height can be re-set based on the observed distance between the edge of the cut and the lower end of most of the developing spikes on the main tillers. Hence, if in a sample of 5 to 10 male plants, an average aboveground height of 5 cm is measured, the cutting test on the test area uses 5 cm as cutting height, and then the cut plants are checked to see if most of the main spikes were cut, or the cut plants are checked to determine the new cutting height so that most main spikes are cut (and if an average aboveground height of 10 cm is measured, the cutting test on the test area uses 10 cm as test cutting height). In another embodiment, the cutting height in a test cutting on a test area of the male plants can be obtained by deducting the sowing depth from the average aboveground height as defined herein, and to use that as test cutting height (e.g., if the average aboveground height was 7 cm and the sowing depth was 2 cm, then the cutting height is set at 5 cm).

Preferably, the cutting tool is moveable. In particular, it is motor driven or supported by a motor driven vehicle. In a preferred embodiment, the cutting tool is a mowing machine, a reaping machine, or a mulching machine. In particular, the cutting tool is a mechanical mowing machine, mechanical reaping machine, or a mechanical mulching machine. The cutting height, i.e. the height at which the tool cuts, is adjustable.

The cutting tool is however not limited to the above mechanical cutting tools. Alternatively, the cutting step can be carried out manually. Thus, the cutting tool may be a knife or a sickle. Moreover, it is envisaged to use scissors as a cutting tool.

In a preferred embodiment, the cutting tool is mowing machine. A preferred mowing machine is a lawn mower.

The cutting tool, in some embodiments, has a mulching function. For examples, it is a mulching lawn mower.

As a result of the cutting step, a portion of the plants has been cleaved off the plants. Thus, this step results in the formation of the debris. In an embodiment of the present invention, the debris that results from the step, i.e. the cut plant material, is removed from the field. In a more preferred embodiment, the debris it is not removed from the field, i.e. it is not collected and not removed from the field. Thus, the cut plant material is left on the soil. Here, it may act as fertilizer and could protect the soil from drying-out. In some embodiments, the debris is blown by the cutting tool (e.g. mulching lawn mower) between male and female area.

Optionally, the field the at least one male area or row is rolled flat before cutting using a standard roll. This would allow for reducing the unevenness of the field (as compared to the unrolled area) and for cutting the plants with similar cutting heights.

### Setting the cutting height

In order to delay flowering/heading of the designated male cereal plants and/or to synchronize flowering/heading, it is advantageous to carry out the cutting step in certain stages (e.g. in Zadoks stages 31 and 32, when the developing spikes (i.e. ears) of the main tillers have a certain aboveground height. For example, the plants can be cut when the average aboveground height of the developing spikes of the main tillers of the designated male cereal plants is at least 4 cm, such as 4 to 15 cm, or 4 to 10 cm.

Thus, in one embodiment, the aboveground height of the developing spike (i.e. ear) of the main tiller of the male plants is a parameter for setting the cutting height of the cutting tool.

In an embodiment, the cutting height of the cutting tool can be set by testing different cutting heights and cutting some of the designated male plants at these (test) cutting height. Afterwards it is assessed whether the plants were cut at a suitable height, for example at a height below the height of the developing spike of the main tiller and above the developing spikes of the secondary tiller, or not. A test cutting height which cuts the plants at the desired height can be used in the method of the present invention.

Alternatively, the parameter can be determined by the determining the aboveground height of the developing spikes of the main tillers. The aboveground height of the developing spike (i.e. ears) of the main tiller of a wheat plant can be determined by measuring the distance from the a) lower end of the crown to b) the upper end (tip) of the developing spike in said plant. For doing so, the entire plant is preferably removed/pulled from the field/soil. Afterwards, a transversal cut through the stem of the harvested plant is made and the distance from the lower end of the crown to the tip of the developing spike is measured. The measured distance is the aboveground height. For determining the average aboveground height, the distance is, preferably, measured in at least 5 plants, such as in at least 10 male plants. In an embodiment, the average aboveground height is determined for each male area with the same male plants. In another embodiment, it is determined for the entire field.

In a preferred embodiment of the methods of the present invention, the methods comprise, prior to the cutting step the step of determining the average aboveground height of the developing spikes of the main tillers of the designated male cereal plants (in the least one area or in the entire field).

Typically, the determination comprises
i) measuring the distance from the a) lower end of the crown to b) the upper end of the developing spike in at least 5 male plants, in particular in at least 10 male plants, and
ii) determining the average aboveground height by calculating the average of the distances measured in step i).

In an embodiment, the plants in step i) are from a single male area.

In another embodiment, the plants in step i) are from different male areas in the same field.

In particular, the determination comprises
i) harvesting at least 5 male plants, in particular in at least 10 male plants,
ii) making a transversal cut through the main stem of each of the plants harvested in step i),
iii) measuring the distance from the a) lower end of the crown to b) the upper end of the developing spike for each of the plants by using the transversal cut, and
iv) determining the average aboveground height by calculating the average of the distances measured in step iii).

In an embodiment, the plants in step i) are from a single male area. In another embodiment, the plants in step i) are from different male areas

The choice of the aboveground height of the developing spikes of the main tillers as a parameter has the following benefits:
- The cutting height depends on the height of the plants to be cut. This gives the farmer some flexibility as the plants can be cut at different stages, rather than at an exact time point.
- It allows for deciding on a time point for the cutting step. Advantageously, the cutting step is carried out, if the average aboveground height of the developing spike of the male cereal plants is in certain range, e.g. in a range from about 5 cm to 15 cm, in particular in a range from about 7 cm to 12 cm. If the male plants have not yet reached this height, they could be allowed to grow further until they reach this height. At this height, the male plants could be cut to delay heading/flowering, while developing a sufficient number of spikes to pollinate the female plants. Further, the plants reach a sufficient height for pollinating the plants.
- It allows for setting the cutting height of the cutting tool of the method of the present invention.

As used herein, when referring to a cutting height of "about" x cm or when referring to an average aboveground height, as used herein, of "about" x cm, this includes values that are 20%, 15% or 10% lower or higher than the given value, to account for expected variation. Hence, a cutting height of about 5 cm as used herein refers to a cutting height of 4-6 cm, and an average aboveground height of about 10 cm refers to an average aboveground height of 8-12 cm.

In one embodiment, after cutting additional nitrogen can be applied to the field or to the male plant areas, so as to stimulate plant recovery. Also, irrigation of the cut plants can help with plant recovery.

### The plants grown on the field

As set forth above, the field provided in step a) of the present invention comprises a population of designated male cereal plants (herein also referred to as "male plants") and a population of designated male-sterile female cereal plants (herein also referred to as "female plants"). A male parent (or pollen parent), is a parent plant that provides the male gametes (pollen) for fertilization, while a female parent or seed parent is the plant that provides the female gametes for fertilization, said female plant being the one bearing the desired hybrid seeds.

The methods of the present invention are, in particular, advantageous for applications which require the crossing of cereal plants which are difficult to cross, such as wheat and barley. For example, the methods can be applied to plants which will be used for wheat hybrid seed production (i.e. for preparing of a cross of male x female (male sterile plants) and for male-sterile plant seed production/maintenance (crosses of between line A wheat plants and line B wheat, where the B is very similar to the A line but is fertile (near-isogenic)). Advantageously, the cutting down approach can be used for any hybrid system, including a cytoplasm-based 3-line hybrid system or a nuclear genic 2-line hybrid system, or a chemically-induced male sterility system using a chemical hybridization agent (CHA, such as sintofen or clofencet).

The designated female cereal plants that are grown on the field, i.e. the pollen acceptors, are, preferably, male sterile plants.

The term "male sterility" or "male sterile" in connection with the present invention refers to the failure or partial failure of plants to produce functional pollen or male gametes. This can be due to natural or artificially introduced genetic predispositions or to human intervention on the plant in the field. In one embodiment, the male sterile plants are genetic cytoplasmatic sterile plants (CMS plants). In another embodiment, the male sterile plants are genetic male sterile plants (GMS plants). Alternatively, the female plants are conditionally male-sterile plant, i.e. to a plant which under normal growing conditions is male fertile and which can be made male-sterile under specific conditions. Also, a plant can be made male-sterile by treating the female plant with a chemical hybridization agent (CHA, such as sintofen). The treatment with the chemical hybridization agent can be done before or after the cutting, according to the recommendations for the CHA used. Preferably, the female plants are treated only.

In a cytoplasmic male sterile (CMS) system, the male sterile (A-line) is crossed to a maintainer line (B-line), which has the almost identical nuclear genotype but is fertile. The maintainer line carries recessive restorer alleles (rf); therefore, when this male fertile line is crossed to a sterile CMS plant, it creates sterile progeny. For commercial hybrid seed production, a male sterile line must be crossed to a line carrying dominant restorer alleles, or restorer line. This is necessary for producing fertile F1 seed. A functional restorer gene for wheat G-type cytoplasmic male sterility encodes a polypeptide which allows for restoring cytoplasmic male sterility (abbreviated "CMS").

With "genetic male sterility (GMS)", nuclear male sterility (Ms) genes control the male sterility condition. A homozygous mutation of the male fertility gene leads to male sterility of wheat plants (in the absence of a male fertility restorer gene). This can be achieved by inactivating or deleting the wheat MS1, MS5, MS9, MS22, MS26, and/or MS45 gene(s) or other genes critical for male gamete/pollen production. In one embodiment, all related sub-genome versions of wheat male fertility genes are inactivated/deleted (i.e. on each wheat sub-genome (A, B and D)). A male fertility restorer gene, typically, restores the male sterility of the plant caused by the homozygous mutation, such as the deletion or inactivation, of the male fertility gene. Preferably, the male fertility restorer gene is a dominant gene. The male fertility restorer gene may be a MS1, MS5, MS9, MS22, MS26, or MS45 gene, specifically depending on what causes the male sterility. For example, if the (homozygous) inactivation or deletion of the endogenous MS1 gene(s) causes male sterility, then a MS1 gene typically, is the restorer gene, and if the (homozygous) inactivation or deletion of the endogenous MS45 genes causes male sterility, then a MS45 gene, typically, is the restorer gene.

Cutting the male plants will reduce the height of the male plants relative to the uncut male plants, and can reduce the height of the male plants relative to the female plants. As known by the skilled person, it would allow for an improved pollination, if the flowering male plants are taller than the female plants or if the female plants have about the same height as the male plants. For example, it was shown that improved pollen diffusion occurs from taller male plants to shorter female plants (Boeven et al., 2016. Genetic architecture of male floral traits required for hybrid wheat breeding. Theoretical and Applied Genetics, 129, pp. 2343-2357).

In a preferred embodiment of the present invention, the designated female cereal plants are semidwarf plants, or the designated male plants are genetically tall plants, so that the designated female plants are shorter than the male plants. Alternatively or additionally, the female plants are treated with a plant growth regulator that reduces the height of said plants.

Dwarf male-sterile female cereal plants are known in the art and are described of Yang et al., (2009, A revolutionary Breeding Approach to wheat, Q.Y. Shu (ed), Induced Plant Mutations in the Genomics Era. Food and agriculture organization of the United Nations, Rome, p370-372, on the world wide web at: www.fao.org/docrep/012/i0956e/l0956e.pdf). Most dwarf lines contain Rht (Reduced height) alleles, for example Rht1 or Rht2 alleles (Horgan et al., 2021. Seedling elongation responses to gibberellin seed treatments in wheat. Agrosystems, Geosciences & Environment, 4(1), p.e20144). In one embodiment, the female plants comprise one or more GA (gibberellin) insensitive Rht alleles.

The term "dwarf plant" includes dwarf, semi-dwarf and double-dwarf plants. Preferred dwarf plants and known dwarfing (Rht) genes are disclosed in Würschum et al. 2017 (The Plant Journal 92, 892-903), Song et al. 2023 (Theor. Appl. Genet. 136(3):62), Liu et al. 2024 (Theor. Appl. Genet. 137, 128) and in WO2015135940A1 which are herewith incorporated by reference with respect to the disclosed dwarf genes/plants.

Plant growth regulators that reduce the height of a plant are well known in the art. For example, trinexapac-ethyl can be used for reducing the height of the female plants. In one embodiment only the female plants are treated with such a growth regulator (e.g. by spraying).

In a preferred embodiment of the present invention, the male plants comprise one or more GA (gibberellin) sensitive Rht alleles, such as the Rht24 allele. Plants with the GA sensitive dwarfing loci will elongate after GA treatment. Thus, the method of the present invention may emcompass the treatment of the male plans with a gibberellin, such as gibberellic acid.

### Effects of the present invention

The cutting step carried out in the method of the present invention has the following effects:
i. the heading/ flowering of the designated male cereal plants is delayed and the flowering period of the designated male cereal plants can be extended (e.g., when some male plants are cut as described herein, and other male plants are not cut) as compared to corresponding control plants that were not subjected to the cutting step in the method of the present invention,
ii. the heading/flowering of the designated male cereal plants and the designated male cereal plants is synchronized, and/or
iii. the seed yield of said field is increased as compared to a corresponding control field that was not subjected to the cutting step in the method of the present invention.

Preferably, the heading/flowering is delayed by at least two days, such by 2 days, 3 days, 4 days or 5 days. For example, the flowering is delayed by 2 to 6 days, in particular by 2 to 5 days, or 2 to 4 days. The delay shall be relative to control plants. Preferably, the control plants were cultivated under the same conditions, but were not subjected to the cutting step. Also preferably, the control plants were grown at the same time on the same field (and were grown in parallel with the plants that were subjected to the cutting step of the invention.

The synchronization is the alignment of heading/flowering timing between the male and female parents. For successful pollination, female receptivity and pollen shed should occur at the same time.

Also, the flowering period of the designated male cereal plants in a field also having male-sterile female plants can be extended by the current invention, e.g., by cutting (one or more) designated male plant row(s) closest to the female plants, and not cutting the remaining male plant row(s) further away from the female plants, so that heading/flowering is delayed for some male plants and not for others, resulting in an extended flowering period.

Moreover, the seed yield of said field can be increased as compared to a corresponding control field that was not subjected to the cutting step. Preferably, the seed yield is the total hybrid seed yield harvested from the female plants only. In an embodiment, the seed yield is increased by at least 10%, such as at least 20%. In another embodiment, the seed yield is increased by at least 50%.

As shown in Example 3, the seed yield is, in particular, increased in the case of negative nicking, i.e. when male plants that were not subjected to the cutting step of the present invention flower before the female plants. Accordingly, the male plants that are grown in accordance with the present invention are preferably plants that are known to head/flower before the female plants (when not subjected to the cutting step). But also when male plants flower around the same time as the female plants, a yield increase can be had on the female plants, and most importantly, as Example 3 shows, surprisingly no significant yield loss was seen in any combination of male and female plants, even when the male plants flower later than the female plants.

### Further subject matter of the present invention

The definitions and explanations provided herein above preferably apply mutatis mutandis to the following:
The present invention also relates to a field obtained or obtainable by the method of the present invention.

Accordingly, the present invention relates to a field comprising one or more areas with designated male cereal plants and one or more areas with designated female cereal plants, wherein the designated male plants in at least part of each male area were cut with a cutting tool during the stem elongation stage and wherein the designated female plants were not cut during the stem elongation stage.

In preferred embodiment, said cereal is wheat, barley, triticale or rye. In particular, the cereal is wheat. Preferably, the plants were sown in the fall.

In a preferred embodiment, the designated male cereal plants were cut in the one-node stage (Zadoks stage 31), two-node stage (Zadoks stage 32) or three-node stage (Zadoks stage 33), in particular the designated male cereal plants are in the one-node stage (Zadoks stage 31) or two-node stage (Zadoks stage 32).

Preferably, the cutting step removed the developing spike of the main tiller from at least a portion of the plants over which the cutting tool was passed. More preferably, the plants were cut at a height equal to or slightly below the average aboveground height of the developing spikes of the main tillers. Even more preferably, the cutting height was up to 25% lower, such as up to 10% lower than the average aboveground height of the developing spikes of the main tillers. Most preferably, the cutting height was below the average aboveground height of the developing spikes of the main tillers, but above the average height of the developing spikes of the secondary tillers. Typically, the cutting step removes most of the developing spikes on the main tillers of the plants over which the tool is passed, but does not remove most of said developing spikes of the secondary tillers.

In a preferred embodiment, the field comprises at least three areas with designated male-sterile female cereal plants (female areas) and at least three areas with designated male cereal plants (male areas), wherein the male areas and the female areas alternate. Preferably, the distance between the female areas and the adjacent male areas allows for a pollination of the designated female cereal plants by the pollen of the designated male parent plants.

In one embodiment, the cutting tool has been passed over the complete area of each of the male areas.

In another embodiment, the cutting tool has been passed only over a part of each of said one male areas. Said part is preferably a part which is adjacent to a female area. Said part is an area of 20% to 80% of the complete area of the at least one male area, such as an area of 30% to 70%, or 40 to 60 %, or 50 %, of the complete area of the at least one male area.

In a preferred embodiment, the debris that resulted from the cutting step was not removed from the field.

In an embodiment of the field of the present invention, the plants are not yet flowering.

Finally, the present invention relates to the use of a cutting tool for selectively cutting designated male cereal plants during the stem elongation stage that are grown on a field, said field comprising at least one area with designated male cereal plants and at least one area with designated male-sterile female cereal plants.

The term "cutting tool" has been defined herein above. In an embodiment, the cutting tool is a lawn mower. The term "selectively cutting" means that only plants in the male areas are cut, wherein as plants in the female area are not cut.

All patents, patent applications, and publications or public disclosures referred to or cited herein are incorporated by reference in their entirety.

The invention will be further described with reference to the examples described herein; however, it is to be understood that the invention is not limited to such examples.

In the following, preferred items of the invention are summarized. The definitions and explanations provided above apply mutatis mutandis.
1. A method for cultivating a field comprising one or more areas with designated (fertile) male cereal plants (male areas) and one or more areas with designated (male sterile) female cereal plants (female areas), said method comprising the step of passing, during the stem elongation stage of the designated male plants, a cutting tool over at least a part of each of said one or more male areas, but not over one or more female areas, to cut the designated male cereal plants in said part of each of said one or more male areas.
2. The method of item 1, wherein said cereal is wheat, barley, triticale or rye.
3. The method of item 2, wherein said wheat is winter wheat.
4. The method of any one of items 1 to 3, wherein the designated male cereal plants are in the one-node stage (Zadoks stage 31), two-node stage (Zadoks stage 32) or three-node stage (Zadoks stage 33), in particular wherein the designated male cereal plants are in the one-node stage (Zadoks stage 31) or two-node stage (Zadoks stage 32).
5. The method of any one of items 1 to 4, wherein the cutting step delays the heading of the cut designated male plants as compared to uncut control plants by about two to six days, such as by about two to four days.
6. The method of any one of items 1 to 5, wherein the cutting step removes the developing spike of the main tiller from at least a portion of the plants over which the cutting tool is passed.
7. The method of any one of items 1 or 6, wherein the cutting tool has a preselected cutting height.
8. The method of item 7, wherein the cutting height is a height which is equal to or slightly below the average aboveground height of the developing spikes of the main tillers.
9. The method of item 7 or 8, wherein the cutting height is up to 25% lower, such as up to 10% lower than the average aboveground height of the developing spikes of the main tillers.
10. The method of any one of items 7 to 10, wherein the cutting height is below the average aboveground height of the developing spikes of the main tillers, but above the average aboveground height of the developing spikes of the secondary tillers, preferably so that most secondary tillers are not cut.
11. The method of any one of items 1 to 10, wherein the cutting tool removes most of the developing spikes on the main tillers of the plants over which the tool is passed, but does not remove most of the developing spikes of the secondary tillers.
12. The method of any one of the preceding items, wherein the average aboveground height of the developing spikes of the main tillers of the designated male cereal plants is at least 4 cm.
13. The method of any one of the preceding items, wherein the average aboveground height of the developing spikes of the main tillers of the designated male cereal plants is from 4 to 15 cm.
14. The method of any one of the preceding items, wherein the method comprises prior to the cutting step a step of determining the average aboveground height of the developing spikes of the main tillers of the designated male cereal plants to set the cutting height at which the cutting tool cuts the designated male cereal plants.
15. The method of item 14, wherein the determination of the average aboveground height of the developing spikes of the main tillers comprises:
   i) measuring the distance from the a) lower end of the crown to b) the upper end of the developing spike in at least five male plants, in particular in at least ten male plants, and
   ii) determining the average aboveground height by calculating the average of the distances measured in step i).
16. The method of item 14 or 15, wherein the determination of the average aboveground height of the developing spikes of the main tillers comprises:
   i) harvesting/pulling at least five male plants, in particular at least ten male plants,
   ii) making a transversal cut through the main stem of each of the plants harvested in step i),
   iii) measuring, in the cuts obtained in step ii), the distance from the a) lower end of the crown to b) the upper end of the developing spike for each of the plants, and
   iv) determining the average aboveground height by calculating the average of the distances measured in step iii).
17. The method of any one of the preceding items, wherein the field comprises at least three areas with designated female cereal plants (female areas) and at least three areas with designated male cereal plants (male areas), wherein the male areas and the female areas alternate.
18. The method of any one of the preceding items, wherein the distance between the female areas and the adjacent male areas allows for a pollination of the designated female cereal plants by the pollen of the designated male parent plants.
19. The method of any one of the preceding items, wherein the cutting tool is passed over the complete area of each of the male areas.
20. The method of any one of the preceding items, wherein the cutting tool is passed only over a part of each of said one male areas, wherein said part is preferably the part adjacent to a female area.
21. The method of item 20, wherein said part is an area of 20% to 80% of the complete area of the at least one male area, such as an area of 30% to 70% of the complete area of the at least one male area.
22. The method of any one of the preceding items, wherein the cutting tool is a mechanical mowing machine, such as a lawn mover or a mechanical reaping machine.
23. The method of any one of the preceding items, wherein the debris that results from the cutting step is not removed, and wherein optionally the debris is mulched.
24. The method of any one of the preceding items, wherein the designated male plants are removed or destroyed before they set seed.
25. The method of any one of the preceding items, wherein
   - the heading of the designated male cereal plants is delayed as compared to corresponding control plants that were not subjected the cutting step
   - the flowering time/window of the male plants is enlarged
   - the heading of the designated male cereal plants and the designated male cereal plants is synchronized, and/or
   - the seed yield of said field is increased as compared to a corresponding control field that was not subjected to the cutting step.
26. The method of any one the preceding items, wherein the female cereal plants are semi-dwarf plants and/or wherein the designated female cereal plants are (selectively) treated with a plant growth regulator that reduces the height of said plants.
27. The method of any one the preceding items, wherein the designated male cereal plants are (selectively) treated with a plant growth regulator that increases the height of said plants, and/or wherein the designated male cereal plants are genetically taller than the designated female cereal plants.
28. The method of any one of items 1 to 27, wherein the designated female cereal plants are male sterile plants.
29. The method of item 28, wherein the male sterile plants are selected from:
   a) cytoplasmic male sterile plants
   b) gen(et)ic male sterile plants,
   c) conditionally male sterile plants, or
   d) male sterile plants that are obtained by treating the designated female plants with one or more chemical hybridization agents.
30. The method of any one of the preceding items, wherein the designated male cereal plants and the designated female cereal plants are genetically diverse.
31. The method of any one of the preceding items, wherein the designated male cereal plants and the designated female cereal plants are nearly isogenic.
32. A field comprising one or more areas with designated male cereal plants and one or more areas with designated female cereal plants, wherein the designated male plants in at least part of each male area were cut with a cutting tool during the stem elongation stage and wherein the designated female plants were not cut during the stem elongation stage.
33. Use of a cutting tool for selectively cutting designated male cereal plants during the stem elongation stage that are grown on a field, said field comprising one or more areas with designated male cereal plants and one or more areas with designated female cereal plants.
34. Any one of the methods in the above items, or the field above, wherein the male areas are blocks/strips with male plant rows, separated from the female areas which are blocks/strips with (male-sterile) female plant rows.

Possible further embodiments of the current invention are reflected in the following numbered paragraphs:
1. Use of a cutting tool for selectively cutting designated male cereal plants growing on a field during the stem elongation stage, said field comprising one or more areas with designated male cereal plants (male areas) and one or more areas with designated male-sterile female cereal plants (female areas), wherein the cutting tool passes over at least a part of each of said one or more male areas, but not over one or more female areas, to cut the designated male cereal plants in said part of each of said one or more male areas.
2. The use of paragraph 1, wherein said cereal is wheat, barley, triticale or rye, such as winter wheat.
3. The use of paragraph 1 or 2, wherein the designated male cereal plants are in the one-node stage (Zadoks stage 31), two-node stage (Zadoks stage 32) or three-node stage (Zadoks stage 33), in particular the designated male cereal plants are in the one-node stage (Zadoks stage 31) or two-node stage (Zadoks stage 32).
4. The use of any one of paragraphs 1 to 3, wherein the cutting step delays the heading of the cut designated male plants as compared to uncut control plants by about two to six days, such as by about two to four days.
5. The use of any one of paragraphs 1 to 4, wherein the cutting step removes the developing spike of the main tiller from at least a portion of the plants over which the cutting tool is passed.
6. The use of any one of paragraphs 1 to 5, wherein the cutting is at a height which is equal to or slightly below the average aboveground height of the developing spikes of the main tillers.
7. The use of paragraph 6, wherein the cutting height is below the average aboveground height of the developing spikes of the main tillers, but above the height of the developing spikes of the secondary tillers, so that most secondary spikes are not cut.
8. The use of any one of paragraphs 1 to 7, wherein the cutting tool removes most of the developing spikes on the main tillers of the plants over which the tool is passed, but does not remove most of the developing spikes of the secondary tillers.
9. The use of any one of the preceding numbered paragraphs, wherein the average aboveground height of the developing spikes of the main tillers of the designated male cereal plants is at least 4 cm.
10. The use of any one of the preceding numbered paragraphs, wherein prior to the cutting the average aboveground height of the developing spikes of the main tillers of the designated male cereal plants is determined to set the cutting height at which the cutting tool cuts the designated male cereal plants, and said determination of the average aboveground height comprises:
   i)measuring the distance from the a) lower end of the crown to b) the upper end of the developing spike in a transversal cut through the main stem of at least five male plants, in particular at least ten male plants, and
   ii)determining the average aboveground height by calculating the average of the distances measured in step i).
11. The use of any one of the preceding numbered paragraphs, wherein :
   i) the cutting tool is passed only over a part of each of said male areas, and wherein said part is the part adjacent to a female area, or
   ii) the cutting tool is a mechanical mowing machine, such as a lawn mower or a mechanical reaping machine, or
   iii) the debris that results from the cutting step is not removed, and wherein optionally the debris is mulched, or
   iv) the designated male plants are removed or destroyed before they set seed, or
   v) before cutting all male areas to be cut, the cutting tool is passed over a test area (such as passing the cutting tool over 1-3 m or 1-3 m² of male plants) to determine if the intended cutting height removes most of the developing spikes on the main tillers, but not most of the developing spikes on the secondary tillers, and adjusting the cutting height as needed,
   vi) the male areas are blocks/strips with male plant rows, separated from the female areas which are blocks/strips with (male-sterile) female plant rows,
   vi) the cutting height is set at 5-7 cm when the measured aboveground height of the spike on the main tiller is 5-7 cm.
12.The use of any one of the preceding numbered paragraphs, wherein :
   - the heading of the designated male cereal plants is delayed as compared to corresponding control plants that were not subjected to the cutting step,
   - the flowering window of the designated male cereal plants is enlarged,
   - the heading of the designated male cereal plants and the designated male cereal plants is synchronized, and/or
   - the seed yield of said field is increased as compared to a corresponding control field that was not subjected to the cutting step.
13.The use of any one the preceding numbered paragraphs, wherein the designated male cereal plants are responsive to gibberellic acid and are treated with gibberellic acid that increases the height of said plants, particularly the cut male plants.
14. A field comprising one or more areas with designated male cereal plants (male areas) and one or more areas with designated male-sterile female cereal plants (female areas), wherein the designated male plants in at least part of each male area were cut with a cutting tool during the stem elongation stage and wherein the designated female plants were not cut during the stem elongation stage.
15. The field of paragraph 14, wherein said cereal is wheat, barley, triticale or rye, such as winter wheat.
16. The field of paragraph 14 or 15, wherein at the time of cutting the designated male cereal plants were in the one-node stage (Zadoks stage 31), two-node stage (Zadoks stage 32) or three-node stage (Zadoks stage 33), in particular the designated male cereal plants were in the one-node stage (Zadoks stage 31) or two-node stage (Zadoks stage 32).
17. The field of any one of paragraphs 14 to 16, wherein the cutting delays the heading of the cut designated male plants as compared to uncut control plants by about two to six days, such as by about two to four days.
18. The field of any one of paragraphs 14 to 17, wherein the cutting removed the developing spike of the main tiller from at least a portion of the plants over which the cutting tool was passed.
19. The field of any one of paragraphs 14 to 18, wherein the cutting was at a height equal to or slightly below the average aboveground height of the developing spikes of the main tillers.
20. The field of paragraph 19, wherein the cutting height was below the average aboveground height of the developing spikes of the main tillers, but above the height of the developing spikes of the secondary tillers, so that most secondary spikes were not cut.
21. The field of any one of paragraphs 14 to 20, wherein the cutting tool removed most of the developing spikes on the main tillers of the plants over which the tool is passed, but did not remove most of the developing spikes of the secondary tillers.
22. The field of any one of the preceding numbered paragraphs, wherein the average aboveground height of the developing spikes of the main tillers of the designated male cereal plants was at least 4 cm.
23. The field of any one of the preceding numbered paragraphs, wherein prior to the cutting the average aboveground height of the developing spikes of the main tillers of the designated male cereal plants was determined to set the cutting height at which the cutting tool cuts the designated male cereal plants, and said determination of the average aboveground height comprised:
   i)measuring the distance from the a) lower end of the crown to b) the upper end of the developing spike in a transversal cut through the main stem of at least five male plants, in particular at least ten male plants, and
   ii)determining the average aboveground height by calculating the average of the distances measured in step i).
24. The field of any one of the preceding numbered paragraphs, wherein :
   i) the cutting tool was passed only over a part of each of said male areas, and wherein said part was the part adjacent to a female area, or
   ii) the cutting tool was a mechanical mowing machine, such as a lawn mower or a mechanical reaping machine, or
   iii) the debris that results from the cutting step was not removed, and wherein optionally the debris was mulched, or
   iv) the designated male plants were removed or destroyed before they set seed, or
   v) the cutting height was set at 5-7 cm when the measured aboveground height of the spike on the main tiller was 5-7 cm.
25.The field of any one of the preceding numbered paragraphs, wherein :
   - the heading of the designated male cereal plants is delayed as compared to corresponding control plants that were not subjected to the cutting step,
   - the flowering window of the designated male cereal plants is enlarged,
   - the heading of the designated male cereal plants and the designated male cereal plants is synchronized, and/or
   - the seed yield of said field is increased as compared to a corresponding control field that was not subjected to the cutting step.
26.The field of any one the preceding numbered paragraphs, wherein the designated male cereal plants are responsive to gibberellic acid and were treated with gibberellic acid that increases the height of said plants, particularly the cut male plants.
27. The field obtained by any of the methods or uses described herein.

### EXAMPLES

### Example 1: Analysis of the effect of the moving growth stage on heading

Four different fertile wheat lines with different heading times (i.e., line 14Q3B1511 (earliness score of 6.2), line 14Q3G0097 (earliness score of 5), line 15Q3B2160 (earliness score of 5.6), and line 17Q3B01610 (earliness score of 6.7)) were subjected to a cutting step at different stages. The aim of this experiments was to assess the delay of heading for one cutting height (the heading that was measured refers to the middle of heading, stage Z55, when the spike on the main tiller emerged 50 % above the flagleaf in 50 % of the plants). The results are shown in Fig. 1. The x-axis shows the distance measured between the crown (or base of the tiller) and the top of the developing spike (ear tip) in the main tiller, which was measured prior to mowing (indicated as GS (for growth stage) in Fig. 1). The y-axis shows the delay in heading (HD, in days). The cutting height of the lawn mower was set at 5 cm from the ground. It can be seen that cutting at 5 cm resulted in delayed heading, which delay got longer when the average aboveground height of the spike on the main tiller was higher. E.g., cutting at 5 cm when the average aboveground height of the main spike was about 5 cm (4-6 cm) resulted in a delay of heading of 2-5 days in the 4 different lines, while cutting at 5 cm when the average aboveground height of the main spike was about 12-13 cm resulted in a heading delay of 9-12 days.

### Example 2: Testing the effect of different cutting heights at three different growth stages on heading, spike formation and plant height

14 different pollinators lines of winter wheat (with a fertile cytoplasm, B lines) were sown in separate plots in the fall. At three different stages (1 node, 1 to 2 node, and 2+), the aboveground height of the developing spike (i.e. ear) of the main tiller of the plants was determined by measuring the spike tip to crown distance in transversal cuts of a sample of 10 plants harvested from the plots. The results are as follows (showing the aboveground height measured from crown to spike tip on the main tiller in the different stages in the different wheat lines):
- M1 (one node): 4 - 7,1 cm
- M2 (one node): 4 - 7,5 cm
- M3 (1-2 node): 7,4 - 9,3 cm
- M4 (1-2 node): 7,5 - 9,7 cm
- M5 (2+ node): 10,3 - 11,9 cm
- M6 (2+ node): 10,9 - 11,9 cm

While the intention was to have the M1 and M2 plants in the stage of about 5 cm aboveground height, to have the M3 and M4 plants and in the stage of about 7 cm aboveground height, and to have the M5 and M6 plants in the stage of about 10 cm aboveground height, as indicated in Fig. 2, there was some variation, and the plants were a bit more advanced as can be seen above, but still each of M1-M2, M3-M4 and M5-M6 were in another/later stage, which was the intention.

Plants at each stage were cut at two different heights using a lawn mower (M1 and M3 at 5 cm, M2, M4 and M5 at 7cm, M6 at 10 cm, see also Figure 2). The impact of the cutting step on the delay of heading, the spike formation (ears per m²) and the plant height as compared to uncut control plants was assessed. The results are shown in Figs. 3, 4 and 5 and in Table 1 below. While there was variation in the different lines, it was found that cutting always resulted in a heading delay of at least 2 days, on average 4-6 days. There was on average a spike reduction of 22 to 35 %, which was less severe when cutting in the earlier growth stages. Also plant height reduced by 12 to 20 % on average, with less height reduction when cutting in the earlier growth stages. As can be seen in Table 1, to get an average delay of about 5 days, plants in later stages are best cut at a higher cutting height (e.g., for a heading delay of about 5 days cutting at 5 cm is best for plants in the 1 node stage, cutting at 7 cm is best for plants in the 1-2 nodes stage, and cutting at 10 cm is best for the plants in the 2+ nodes stage (each stage having the above measured aboveground height at each stage)). Also, Table 1 show that cutting later stages results in an increase in the reduction in spikes and an increase in the reduction in plant height.

**Table 1: summary of the results for all lines tested**

| | | **Cutting height** | | |
|---|---|---|---|---|
| **Cutting time** | | **5cm** | **7cm** | **10cm** |
| **Average of Delta heading (days)** | | | | |
| | 1node | 5.5 | 4.1 | |
| | 1-2nodes | 5.7 | 4.4 | |
| | 2+nodes | | 5.9 | 4.9 |

| **Average of Spike reduction** | | | | |
|---|---|---|---|---|
| | 1node | 25.7 | 22.0 | |
| | 1-2nodes | 31.7 | 25.6 | |
| | 2+nodes | | 34.7 | 31.4 |

| **Average of Height Reduction** | | | | |
|---|---|---|---|---|
| | 1node | 14.2 | 12.7 | |
| | 1-2nodes | 16.7 | 15.2 | |
| | 2+nodes | | 19.5 | 17.4 |

### Example 3: Effect of the male cutting step on seed set of female (male sterile) plant

Six different cytoplasmic male sterile female plant lines (A lines) and two different male plant lines (R lines) for hybrid seed production were grown using a strip planting approach. The plants are derived from seeds sown in fall. Female plot dimensions were 1.5m wide x 1.64m long with 6 rows. An overview of the field trial design scheme is shown in Fig. 7. Sowing density was around 130 seeds/m². The measurement (from the crown up to the tip of the developing spike in the main tiller) was done on 10 plants per genotype, taken at random in the male row strips. In case of a large plot, it is preferred to check at least 2 zones to see if there are heterogeneities. Mowing with a lawn mower (set at a cutting height of about 10 cm) was triggered when the distance from the root to the ear was about 10 cm. Before completely mowing the strip, a test over 2-3 meters was done to observe if the ear in most of the main tillers is well cut but not that in most of the secondary tillers. As a control, some male plots were not subjected to the cutting step. The plants were allowed to cross-pollinate the male-sterile female plants. Later, the seeds were harvested from the female rows.

The mowing resulted in a delay of heading by 3 to 4 days, around 35% spike reduction and a height reduction of 15 to 20% in both male lines.

As regards to the seed set in the female plants an increase of 106% to 199% (as compared to the controls) was observed when there was negative nicking, and an increase of 18% to 33% was observed when there was no nicking. When there was positive nicking, the change in the seed set in the female plants was from -8.5% (not significantly different from control) to +73% (see Fig. 6A and 6B).

## Claims

1. A method for cultivating a field comprising one or more areas with designated fertile male cereal plants (male areas) and one or more areas with designated male-sterile female cereal plants (female areas), said method comprising the step of passing, during the stem elongation stage of the designated male plants, a cutting tool over at least a part of each of said one or more male areas, but not over one or more female areas, to cut the designated male cereal plants in said part of each of said one or more male areas.

2. The method of claim 1, wherein said cereal is wheat, barley, triticale or rye, such as winter wheat.

3. The method of any one of claims 1 or 2, wherein the designated male cereal plants are in the one-node stage (Zadoks stage 31), two-node stage (Zadoks stage 32) or three-node stage (Zadoks stage 33), in particular the designated male cereal plants are in the one-node stage (Zadoks stage 31) or two-node stage (Zadoks stage 32).

4. The method of any one of claims 1 to 3, wherein the cutting step delays the heading of the cut designated male plants as compared to uncut control plants by about two to six days, such as by about two to four days.

5. The method of any one of claims 1 to 4, wherein the cutting step removes the developing spike of the main tiller from at least a portion of the plants over which the cutting tool is passed.

6. The method of any one of claim 1 to 5, wherein the cutting is at a height which is equal to or slightly below the average aboveground height of the developing spikes of the main tillers.

7. The method of claim 6, wherein the cutting height is below the average aboveground height of the developing spikes of the main tillers, but above the height of the developing spikes of the secondary tillers, so that most secondary spikes are not cut.

8. The method of any one of claims 1 to 7, wherein the cutting tool removes most of the developing spikes on the main tillers of the plants over which the tool is passed, but does not remove most of the developing spikes of the secondary tillers.

9. The method of any one of the preceding claims, wherein the average aboveground height of the developing spikes of the main tillers of the designated male cereal plants is at least 4 cm.

10. The method of any one of the preceding claims, wherein the method comprises prior to the cutting step a step of determining the average aboveground height of the developing spikes of the main tillers of the designated male cereal plants to set the cutting height at which the cutting tool cuts the designated male cereal plants, and said determination of the average aboveground height comprises:
i)measuring the distance from the a) lower end of the crown to b) the upper end of the developing spike in a transversal cut through the main stem of at least five male plants, in particular at least ten male plants, and
ii)determining the average aboveground height by calculating the average of the distances measured in step i).

11. The method of any one of the preceding claims, wherein :
i) the cutting tool is passed only over a part of each of said male areas, and wherein said part is the part adjacent to a female area, or
ii) the cutting tool is a mechanical mowing machine, such as a lawn mower or a mechanical reaping machine, or
iii) the debris that results from the cutting step is not removed, and wherein optionally the debris is mulched, or
iv) the designated male plants are removed or destroyed before they set seed, or
v) the cutting height is set at 5-7 cm when the measured aboveground height of the spike on the main tiller is 5-7 cm.

12. The method of any one of the preceding claims, wherein :
• the heading of the designated male cereal plants is delayed as compared to corresponding control plants that were not subjected to the cutting step,
• the flowering window of the designated male cereal plants is enlarged,
• the heading of the designated male cereal plants and the designated male cereal plants is synchronized, and/or
• the seed yield of said field is increased as compared to a corresponding control field that was not subjected to the cutting step.

13. The method of any one the preceding claims, wherein the designated male cereal plants are responsive to gibberellic acid and are treated with gibberellic acid that increases the height of said plants, particularly the cut male plants.

14. A field comprising one or more areas with designated male cereal plants and one or more areas with designated male-sterile female cereal plants, wherein the designated male plants in at least part of each male area were cut with a cutting tool during the stem elongation stage and wherein the designated female plants were not cut during the stem elongation stage.

15. Use of a cutting tool for selectively cutting designated male cereal plants during the stem elongation stage that are grown on a field, said field comprising one or more areas with designated male cereal plants and one or more areas with designated male-sterile female cereal plants.
